# EUROPÄISCHE PATENTSCHRIFT

(11) **EP 0 400 108 B1**
(45) Veröffentlichungstag und Bekanntmachung des Hinweises auf die Patenterteilung: **02.01.1997**
(21) Anmeldenummer: 89911524.0
(22) Anmeldetag: 22.10.1989
(51) Int. Cl.: A61B 1/00

(54) **Endoskop**
ENDOSCOPE
ENDOSCOPE

(30) Priorität: 27.10.1988 DE 3836649; 18.04.1989 DE 3912720
(43) Veröffentlichungstag der Anmeldung: 05.12.1990
(73) Patentinhaber: LEMKE, Rosemarie, D-82178 Puchheim (DE); LEMKE, Norbert, D-82178 Puchheim (DE)
(72) Erfinder: LEMKE, Norbert, D-8031 Puchheim (DE)
(74) Vertreter: Münich, Wilhelm, Dr.
(86) Internationale Anmeldenummer: DE8900676
(87) Internationale Veröffentlichungsnummer: WO9004350

(56) Entgegenhaltungen:
- EP-A- 0 100 517
- EP-A- 0 260 855
- DE-A- 2 948 564
- GB-A- 2 148 526
- US-A- 3 417 745

## Beschreibung

### Technisches Gebiet

Die Erfindung bezieht sich auf ein Endoskop mit einem am distalen Ende angeordneten Endoskopobjektiv, dessen Bild eine Bildleiter-Einheit zum proximalen Ende leitet, an dem eine Videokamera abnehmbar angebracht ist, und deren Bildaufnehmer das von der Bildleiter-Einheit weitergeleitete Bild aufnimmt, und einer Beleuchtungseinrichtung, die einen Lichtleiter aufweist, der das Licht von einer Lichtquelle zum distalen Ende leitet, und der die Bildleiter-Einheit ringförmig umgibt.

### Stand der Technik

Derartige Endoskope werden gegenwärtig sowohl im medizinischen als auch im technischen Bereich in einer Vielzahl von Anwendungen eingesetzt. Die derzeit standardmäßig angebotenen Endoskope weisen ein am distalen Ende angeordnetes Endoskopobjektiv auf, dessen Bild eine Bildleiter-Einheit zum proximalen Ende leitet, an dem es mittels eines Okulars oder einer Fernsehkamera, die gegenwärtig in der Regel auf das Okular aufgesetzt wird, von einer Bedienungsperson betrachtet werden kann. Die Beleuchtungseinrichtung weist in der Regel einen Lichtleiter auf, der das Licht von einer Lichtquelle zum distalen Ende leitet, und der die Bildleiter-Einheit ringförmig umgibt.

Zur Einkoppelung des zur Beleuchtung des Hohlraums, in den das Endoskop eingesetzt ist, erforderlichen Beleuchtungslichtes weisen die bekannten Endoskope einen seitlichen, weitgehend genormten Lichtleiter-Anschlußstutzen auf, der mit einem Lichtleit-kabel einer Beleuchtungslichtquelle verbindbar ist. Von diesem seitlichen Lichtleiter-Anschlußstutzen führen eine Vielzahl von Lichtleitfasern, die in den Hohlraum zwischem dem Relais-Linsensystem und einem aus Metall gefertigten äußeren Schutzrohr eingelegt sind, zum distalen Ende.

Die Fertigung dieses aus einer Vielzahl von Lichtleit-Fasern bestehenden Lichtleiters ist aus einer Reihe von Gründen aufwendig:

Zum einen müssen die Lichtleit-Fasern "von Hand" beim Zusammenbau des Endoskops "vorgeordnet" werden.

Zum anderen ist es erforderlich, die einzelnen Lichtleit-Fasern entsprechend dem "Blickwinkel" des am distalem Ende befindlichen Objektivs anzuschleifen bzw. umzubiegen, damit die Achse der Beleuchtung mit der optischen Achse des Objektiv wenigstens annähernd übereinstimmt.

Weiterhin haben die bekannten Endoskope den Nachteil, daß aufgrund der Lichtleitfasern sowie gegebenenfalls auch aufgrund von Kittgliedern im Endoskop eine Sterilisierung im Autoklaven nur eingeschränkt möglich ist. Eine Sterilisierung "in Lösung" ist jedoch insbesondere im Hinblick auf Krankheiten wie Aids etc. unzureichend.

Wenn anstelle oder zusätzlich zur normalen Betrachtung des endoskopischen Bildes eine Darstellung dieses Bildes auf einem Monitor gewünscht wird, wird derzeit an die Okularmuschel entweder direkt oder über einen Bildteiler eine Miniaturvideokamera angesetzt, die das "Okular-Bild" in bekannter Weise mittels eines Objektivs und eines Bildaufnehmers aufnimmt.

Diese bekannten Endoskope gemäß dem Oberbegriff des Anspruchs 1 - wie sie beispielsweise aus der DE 37 43 920 A1 bekannt sind - haben nun eine Reihe von Nachteilen:

Zum einen hat die Miniaturvideokamera aufgrund des erforderlichen Objektivs, des Bildaufnehmers usw. immer noch vergleichsweise große Abmessungen; typischerweise betragen die Länge der Kamera 8 bis 10 cm und ihre Querschnittsabmessungen mehrere cm.

Zum anderen verlängert die Fernsehkamera die Baulänge des Endoskops und ändert damit insbesondere die Gewichtsverteilung; dies verschlechtert die Handhabung der gesamten Einheit.

Weiterhin ist der optische Aufwand für die Videokamera vergleichsweise groß, da ein Okularbild abgebildet werden muß.

Aus der EP 0 100 517 ist ein Endoskop mit einem am distalen Ende angeordneten Endoskopobjektiv zu entnehmen, dessen Bild eine Bildleitereinheit zum proximalen Ende leitet, und einer Beleuchtungseinrichtung, die einen Lichtleiter aufweist, der das Licht von einer Lichtquelle zum distalen Ende leitet. Der Lichtleiter umgibt dabei die Bildleitereinheit ringförmig. Der Lichtleiter weist überdies einen aus einem durchsichtigen und optisch klaren Material bestehenden Hohlzylinder auf, der das Objektiv und die Bildleiter-Einheit umgibt und das Licht der Lichtquelle in die ringförmige Stirnfläche des Hohlzylinders einkoppelt. Der Einsatz einer Videokamera und insbesondere die Kombinierbarkeit einer solchen mit dem in der Druckschrift beschriebenen Endoskopvorrichtung ist jedoch nicht vorgesehen.

### Darstellung der Erfindung

Der Erfindung liegt die Aufgabe zugrunde, ein Endoskop mit einem am distalen Ende angeordneten Endoskopobjektiv, dessen Bild eine Bildleiter-Einheit zum proximalen Ende leitet, an dem ein Videokamera anbringbar ist, die das Bild aufnimmt, und einer Beleuchtungseinrichtung, die einen Lichtleiter aufweist, der das Licht von einer Lichtquelle zum distalen Ende leitet, und der die Bildleiter-Einheit ringförmig umgibt, derart weiterzubilden, daß mit einfachen Mitteln und geringem baulichem Aufwand eine Videobild-Darstellung möglich ist.

Weiter soll ein Endoskop mit einem am distalem Ende angeordneten Objektiv, dessen Bild ein Relais-Linsensystem in dem proximalen Bereich weiterleitet, und einem Lichtleiter, der das Relais-Linsensystem und das Objektiv wenigstens teilweise umgibt, und das Beleuchtungslicht einer Beleuchtungslichtquelle von einem Lichteintritts-Anschluß zum distalem Austrittsende leitet, derart weitergebildet werden, daß die Herstell- und der Zusammenbau des Endoskops, insbesondere hinsichtlich des Lichtleiters vereinfacht wird.

Eine erfindungsgemäße Lösung dieser Aufgabe ist mit ihren Weiterbildungen und Ausgestaltungen in den Patentansprüchen gekennzeichnet.

Die Erfindung geht von dem Grundgedanken aus, die "Schnittstelle" zwischen dem eigentlichen Endoskop und dem Bildaufnehmer nicht "hinter das Okular", sondern direkt "hinter die Bildleiter-Einheit", die beispielsweise in an sich bekannter Weise aus Stablinsen oder aus einem Faserbündel bestehen kann, zu verlegen. Da hierdurch der Bildaufnehmer direkt in der proximalen Bildebene der Bildleiter-Einheit angeordnet werden kann, kann auf ein Objektiv, wie es herkömliche Videokameras für die Endoskopie aufweisen, verzichtet werden. Dies reduziert den baulichen Aufwand, vereinfacht die Herstellung und erhöht gleichzeitig den Transmissionsfaktor, so daß mit schwächeren Beleuchtungslichtquellen gearbeitet werden kann oder bei gleicher Beleuchtungsstärke ein helleres Bild erhalten wird.

Vor allem aber kann bei der erfindungsgemäßen Ausbildung die "Videokamera" wesentlich stärker miniaturisiert werden als dies bei herkömlichen Endoskop-Kameras der Fall ist, da die "Kamera" praktisch nur noch aus dem Bildaufnehmer besteht.

Dieser erfindungsgemäße Aufbau des Endoskops hat den weiteren Vorteil, daß das Einkoppeln des Beleuchtungslichtes vereinfacht ist: Hierzu umgibt ein Lichtleiter den Bildaufnehmer und ist derart ausgebildet, daß er beim Ansetzen der Fernsehkamera an das Endoskop mit dem Lichtleiter des Endoskops optisch verbunden ist.

Damit kann auf den bei herkömlichen Endoskopen vorgesehenen seitlichen Einkoppelungs-Stutzen verzichtet werden, der zum einen die Herstellung verteuert und zum anderen häufig zu einer ungleichmäßigen Bildausleuchtung führt.

Dennoch ist es bei dem erfindungsgemäßen Endoskop möglich, weiterhin zusätzlich, aber auch ausschließlich das Endoskop-Bild rein visuell zu beobachten:

Hierzu wird anstelle der Videokamera oder zusätzlich zu dieser mittels eines optischen Bildteilers ein an sich bekanntes Okular an dem "Rumpf-Endoskop" angebracht. Dieses Okular ist typischerweise in gleicher Weise wie die Okulare aufgebaut, die bei herkömlichen Endoskopen fest mit dem Bildleiter-Teil verbunden sind. Insbesondere kann das Okular in bekannter Weise einen seitlichen Einkoppel-Stutzen für das Beleuchtungslicht aufweisen.

Das erfindungsgemäße Video-Endoskop hat darüberhinaus noch den Vorteil, daß die Sterilisierung der einzelnen Elemente erleichtert wird, da das eigentliche Endoskop aus weniger Teilen besteht.

Ferner weist gemäß Anspruch 2 der Lichtleiter einen aus einem durchsichtigen und optisch klarem Material bestehenden Hohlzylinder auf, der das Objektiv und das Relais-Linsen-system umgibt. Ferner ist das Licht der Beleuchtungslichtquelle in die ringförmige Stirnfläche des Hohlzylinders einkoppelbar.

Anstelle einer Vielzahl von Lichtleitfasern, die "mühsam" in den ringförmigen Zwischenraum zwischem dem Relais-Linsensystem und dem äußeren Schutzrohr des Endoskops eingelegt und dann "geordnet" werden müssen, wird ein "einstückiges" Teil verwendet, das als Lichtleiter dient. Die Einkoppelung des Beleuchtungslichts in dieses Teil erfolgt nicht von der Seite über einen Lichtleiter-Stutzen, sondern über die ringförmige Stirnfläche des Lichtleiters.

Damit entfallen die zeitaufwendigen Assemblierungs-Schritte herkömmlicher Endoskope. Vor allem aber hat der erfindungsgemäße Endoskop-Grundteil den Vorteil, daß das Beleuchtungslicht über eine wesentlich größere Fläche als bei den bekannten Endoskopen geleitet wird. Dabei ist insbesondere zu berücksichtigen, daß bei herkömmlichen Endoskopen - auch bei sorgfältiger Montage - immer ein Teil der Lichtleitfasern "tot" ist; damit ist gemeint, daß durch diese Lichtleitfasern kein Licht zum distalem Ende geleitet wird. Wenn derartige "tote" Lichtleitfasern in einem bestimmten Winkelbereich gehäuft auftreten, so ergibt sich bei herkömmlichen Endoskopen eine ungleichmäßige Beleuchtung des Objektfeldes.

Bei dem erfindungsgemäßen Endoskop-Grundteil kann dagegen das Beleuchtungslicht gleichmäßig über die gesamte ringförmige Stirnfläche eingekoppelt werden, so daß sich eine gleichmäßige Ausleuchtung des Objektfeldes ergibt.

Der erfindungsgemäße "einstückige" Lichtleiter kann im Prinzip aus einem beliebigen durchsichtigen, optisch klarem Material, bspw. aus einem optischen Glas gefertigt werden.

Weiterbildungen der Erfindung sind in den Unteransprüchen angegeben:

Gemäß Anspruch 3 ist die Lichtaustrittsfläche des den Bildaufnehmer umgebenden Bildleiters in Richtung der Endoskop-Längsachse von dem in der Bildebene der Bildleiter-Einheit angeordneten Bildaufnehmer beabstandet. Dies hat den Vorteil, daß kein Streulicht von der Verbindungsstelle der beiden Lichtleiter auf den Bildaufnehmer auftreffen kann.

Im Anspruch 4 ist gekennzeichnet, daß zwischen Bildleiter-Einheit und Bildaufnehmer ein zusätzliches Linsenelement vorgesehen ist. Dieses zusätzliche Linsenelement dient zur Größenanpassung des Endoskop-Bildes an die Abmessungen des Bildaufnehmers.

Weiterhin ist bevorzugt, wenn das Kamera-Anschlußkabel für die Videokamera mit dieser mittels eines Steckers verbunden ist, in dem die Lichtleiterkupplung und die Verbindungselemente für die elektrischen Verbindungen einen Abstand senkrecht zur Kabel-Längsachse aufweisen (Anspruch 5). Hierdurch wird verhindert, daß der Lichtleiter die elektrischen Verbindungen sowie gegebenenfalls im Stecker vorgesehene elektrische Elemente zu stark erwärmt.

Hierzu dienen auch die im Anspruch 6 angegebenen Merkmale, gemäß denen die elektrischen Verbindungsleitungen im Kamera-Anschlußkabel den Lichtleiter umgeben.

Als weiterer großer Vorteil ist zu nennen, daß ein getrenntes Lichtleitkabel entfällt, so daß das erfindungsgemäße Endoskop im Gegensatz zu herkömlichen Video-Endoskopen nur noch ein einziges Anschlußkabel aufweist.

Bevorzugt ist es ferner, wenn der als Hohlzylinder ausgebildete Lichtleiter aus einem optisch klarem Kunststoffmaterial (Anspruch 13) besteht. Im Anspruch 14 ist ein mögliches Kunststoffmaterial, nämlich Polycarbonat gekennzeichnet, das u.a. den Vorteil hat, daß es leicht im Spritzgießverfahren verarbeitet werden kann, so daß der erfindungsgemäße Lichtleiter problemlos und kostengünstig als Formteil, herstellbar ist. Bevorzugt ist es aber auch möglich, den erfindungsgemäßen Lichtleiter "als Meterware" aus entsprechenden Rohren herzustellen und gegebenenfalls thermisch nachzubehandeln.

Der Lichtleiter, der einen aus einem durchsichtigen und optisch klarem Material bestehenden Hohlzylinder aufweist, kann insbesondere gemäß Anspruch 8 im proximalen Bereich in einen zylindrischen Abschnitt mit größerem Innen- und Außendurchmesser übergehen. In diesem Falle bildet dann die freie Stirnfläche des Bereichs mit größerem Durchmesser die Licht-Eintrittsfläche für das Beleuchtungslicht.

Diese Ausbildung hat den Vorteil, daß das sich an das Relais-Linsensystem anschließende System zur Bildübertragung bzw. -darstellung einen größeren Durchmesser als das Relais-Linsensystem haben kann.

In jedem Falle ist es jedoch bevorzugt, wenn die als Lichteintrittsfläche dienende Stirnfläche des Lichtleiters in Längsrichtung des Endoskop-Grundteils vor oder hinter der proximalen Bildebene des Relais-Linsensystems liegt (Anspruch 3), da dann Reflexe an der Lichteintrittfläche nicht in die Bildebene gelangen.

Der verwendete Lichtleiter hat gemäß Anspruch 9 den weiteren Vorteil, daß es in einfacher Weise möglich ist, durch eine entsprechende "Abschrägung" der als Lichtaustrittsfläche dienenden Stirnfläche des Lichtleiters das Beleuchtungslicht unter dem Winkel austreten zu lassen, den die optische Achse des "Schrägblick-Objektivs" mit der Längsachse des Endoskop-Grundteils einschließt, so daß mit einem kostengünstigen Bearbeitungsschritt "Koaxialität" zwischen Beleuchtung und Beobachtung gewährleistet ist. Dennoch stellt der erfindungsgemäße Lichtleiter eine homogene Beleuchtung des Objektfeldes sicher.

Gemäß Anspruch 10 sind zwischen dem den Lichtleiter bildenden Hohlzylinder einerseits und dem Objektiv bzw. dem Relais-Linsensystem andererseits ein lichtabsorbierendes und/oder -reflektierendes Element vorgesehen, das den Übertritt von Licht aus dem Lichtleiter in das Relais-Linsensystem und damit das Entstehen von Reflexionen verhindert.

Dieses Element kann gemäß Anspruch 11 bspw. ein Kunststoff-Schrumpfschlauch sein, in den die Elemente des Relais-Linsensystems in der richtigen Position eingesetzt werden, und der anschließend durch Temperatureinwirkung geschrumpft wird. Damit hält dieses Element gleichzeitig auch die Elemente des Relais-Linsensystems, so daß eine "Linsen- bzw. Objektivfassung im herkömmlichen Sinne" überflüssig wird.

Diese Elemente können gemäß Anspruch 13 ebenfalls aus einem optisch klarem Kunststoffmaterial bestehen, das wiederum bspw. spritzgießbares Polycarbonat sein kann (Anspruch 14).

Durch die vorstehend genannten Weiterbildungen werden die Herstellkosten eines herkömmlichen Endoskops derart gesenkt, daß das erfindungsgemäße Endoskop jederzeit auch als Einmal-Endoskop verwendet werden kann. Vergleichbare Endoskope herkömmlicher Bauart haben dagegen gegenwärtig einen Verkaufspreis in der Größenordnung von mehreren Tausend DM, so daß sich eine Anwendung als Einmal-Endoskop von selbst verbietet.

Dennoch kann das erfindungsgemäße Endoskop insbesondere dann, wenn es gemäß Anspruch 15 ein Kunststoff-Formteil aufweist, das ein in an sich bekannter Weise ausgebildetes Flanschelement zum Verbinden mit herkömmlichen Endoskop-Schäften besitzt, jederzeit anstelle von kerkömmlichen Endoskopen in vorhandenen Endoskop-Systemen eingesetzt werden.

Dabei ist weiterhin ein Arbeiten unter Sicht, bspw. mit einem fest mit dem Endoskop-Grundteil verbundenen Okular möglich.

Bevorzugt ist es jedoch, wenn das erfindungsgemaße Endoskop-Grundteil - im Gegensatz zu herkömmlichen Endoskopen - keinen fest an dem Grundteil angebrachten Beobachtungsteil aufweist, sondern wenn mit dem erfindungsgemäßen Endoskop-Grundteil, wahlweise ein Okular oder eine Videokamera verbindbar sind.

Beim Ansetzen eines Okulars ist es bevorzugt, wenn dieses einen an sich bekannten seitlichen Lichtleiter-Eintrittstutzen aufweist, der bspw. über Glasfasern mit einer ringförmigen Beleuchtungslicht-Austrittfläche verbunden ist, die gegenüber der ringförmigen Beleuchtungslicht-Eintrittsfläche des Endoskop-Grundteils angeordnet ist. Trotz dieser Ausbildung, bei der das Okular weitgehend ähnlich wie bekannte Endoskope mit fest angesetztem Okular aufgebaut ist, ergibt sich aufgrund der Trennung von Okular und Endoskop-Grundteil, durch die ein Okular für eine Reihe von Endoskop-Grundteilen verwendet werden kann, eine wesentliche Kosteneinsparung gegenüber herkömmlichen Endoskopen.

Ferner ist es möglich, die Videokamera so auszubilden, daß eine ringförmige Beleuchtungslicht-Austrittsfläche, die den Bildaufnehmer umgibt, derart ausgebildet ist, daß sie gegenüber der ringförmigen Beleuchtungslicht-Eintrittsfläche des Endoskop-Grundteils angeordnet ist.

Das erfindungsgemäße Endoskop eignet sich nicht nur aufgrund seiner kostengünstigen Herstellung als Einmal-Endoskop, das vor Gebrauch bspw. Gamma-sterilisiert wird, sondern es kann aufgrund der (nahezu) vollständigen Fertigung aus Kunststoffmaterial auch durch Mikrowellen-Bestrahlung sterilisiert werden. Dabei ist es bevorzugt, wenn das zu sterilisierende Teil über einer Verdampferschale, die bspw. Sterilwasser enthält, angeordnet ist.

Das durch die Mikrowellen-Bestrahlung verdampfende Wasser schlägt sich (zunächst) auf das "kalt-bleibende" Kunststoffmaterial des Endoskops nieder und löst damit dort vorhandene kristalline Rückstände.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: einen Querschnitt durch ein erfindungsgemäß ausgebildetes Endoskop mit angesetztem Bildaufnehmer,
- Fig. 2: den Verlauf des Anschlußkabels in der Fernsehkamera, und
- Fig. 3: die Ausbildung des Verbindungssteckers,
- Fig. 4: einen Querschnitt durch ein erfindungsgemäß ausgebildetes Endoskop-Grundteil und

### Darstellung von Ausführungsbeispielen

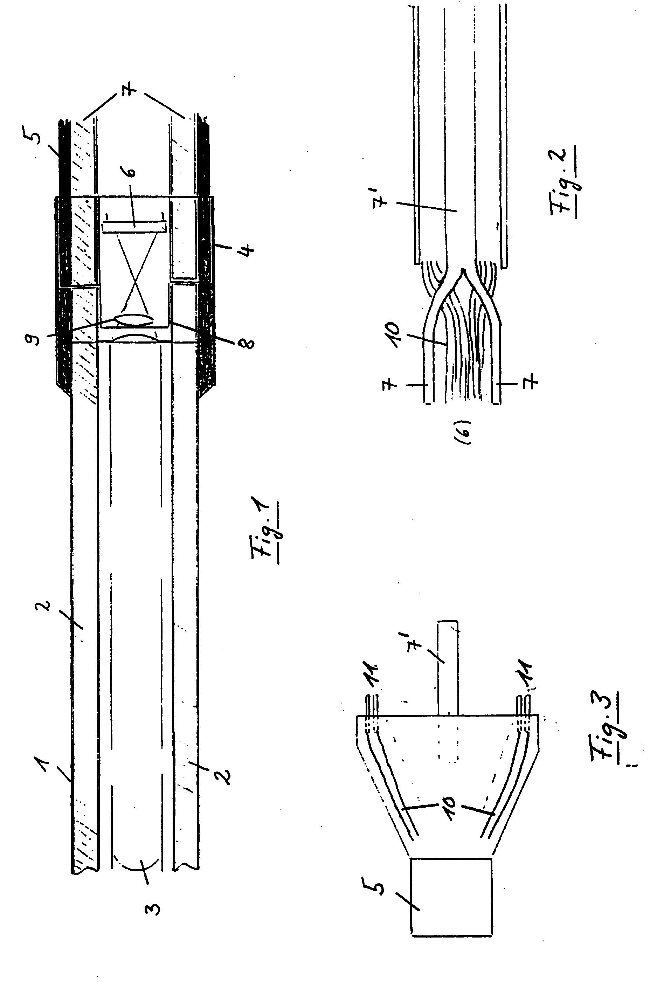

Fig. 1 zeigt ein erstes Ausführungsbeispiel für ein erfindungsgemäßes Endoskop, das in an sich bekannter Weise eine Hülse 1 aufweist, die beispielsweise aus rostfreiem Edelstahl, aber auch aus einem Kunststoffmaterial bestehen kann, und in der ein Lichtleiter 2 sowie eine Bildleiter-Einheit 3 und am - in dieser Figur nicht dargestellten - distalen Ende ein Objektiv vorgesehen sind.

Der Lichtleiter 2 und die Bildleiter-Einheit 3 sind in an sich bekannter Weise aufgebaut: Beispielweise besteht die Bildleiter-Einheit 3 aus Stablinsen-Optiken oder aus einem Faserbündel.

Am proximalen Ende weist das Endoskop neben einer nicht dargestellten, in bekannter Weise ausgebildeten Kupplung zur Verbindung mit einem Endoskop-Schaft ein weiteres Kupplungselement 4 auf, das bei dem gezeigten Ausführungsbeispiel eine Rast-Hülse ist, in der beispielsweise für die Rastung eine nicht dargestellte federbelastete Kugel oder ein O-Ring vorgesehen sind. Mittels dieses Kupplungs-Elements 4 kann eine Videokamera 5 derart mit dem Endoskop verbunden werden, daß sich die lichtempfindliche Fläche eines Bildaufnehmers 6 in der proximalen Bildebene 3' der Bildleiter-Einheit 3 befindet. Ferner weist die Videokamera 5 ein Lichtleit-Element 7 auf, das den Bildaufnehmer 6 ringförmig umgibt und zum Einkoppeln von Beleuchtungslicht in den Lichtleiter 2 des Endoskops dient.

Die Verbindungsstelle zwischen dem Lichtleit-Element 7 und dem Lichtleiter 2 ist in Axialrichtung des Endoskops vom Bildaufnehmer beabstandet und befindet sich bei dem gezeigten Ausführungsbeispiel näher an der Bildleiter-Einheit 3, so daß Streulicht, das an der Verbindungsstelle entstehen kann, von einer Hülse 8 gegenüber dem Bildaufnehmer 6 abgeschirmt wird. Ferner ist eine Linse 9 vorgesehen, die die Größe des proximalen Bildes an die Abmessungen der lichtempfindlichen Fläche des Bildaufnehmers 6 anpaßt.

Fig. 2 zeigt die Ausbildung des Anschlußkabels in der Videokamera:

Im Bereich des Bildaufnehmers 6 liegen die elektrischen Anschlußdrähte 10 innen und werden vom Lichtleiter 7 umgeben. Nach einer kurzen Strecke vereinigt sich der ringförmige bzw. hohlzylindrische Lichtleiter 7 zu einem zylindrischen Lichtleiter 7', während die elektrischen Anschlußdräht 10 nach außen laufen.

Fig. 3 zeigt die Ausbildung eines Verbindungssteckers zwischen der Videokamera 5 und einem nicht näher dargestellten Anschlußkabel. Fig. 3 ist zu entnehmen, daß im Stecker die elektrischen Anschlußdrähte 10 vom Lichtleiter 7' einen größer Abstand haben als beispielsweise in der Kamera. Hierdurch wird erreicht, daß sich die elektrischen Kontaktelemente 11 nicht zu stark erwärmen und somit keine Kontaktprobleme auftreten können.

Das erfindungsgemäße Endoskop ermöglicht nicht nur eine Video-Beobachtung des Endoskop-Bildes, sondern nach Abnahme der Videokamera 5 und Aufstecken eines Okulars auch eine rein visuelle Beobachtung. Das Okular kann dabei in an sich bekannter Weise ausgebildet sein, und insbesondere einen seitlichen Ansatz-Stutzen zum Einkoppeln des Beleuchtungslichts aufweisen.

Darüberhinaus ist es auch möglich, einen Strahlteiler auf das Endoskop aufzustecken, der beispielsweise eine optische Zwischenabbildung ausführt, so daß gleichzeitig eine visuelle Beobachtung und die Aufnahme eines Videobildes möglich sind.

Die erfindungsgemäße Ausbildung hat dabei den besonderen Vorteil, daß in jedem Falle die Zahl der Glasflächen vor dem Bildaufnehmer deutlich geringer als bei herkömlichen Endoskopen ist, so daß der Transmissionsgrad größer und damit das Videobild heller ist.

Weiterhin werden die Gestehungskosten für einen EndoskopSatz, d.h. für Endoskope mit unterschiedlichem Bildwinkel und/oder Gesichtsfeldrichtung deutlich verringert, da für sämtliche Endoskope nur einmal ein Okular angeschafft werden muß, und die erfindungsgemäßen Endoskope, die lediglich aus einem Objektiv, einem Bildleiter und einem Lichtleiter ohne seitliche Einkoppelung bestehen, deutlich kostengünstiger als herkömliche Endoskope herzustellen sind.

Letztlich wird auch die sterilisierbarkeit des in den Körper eingesetzten Endoskops verbessert.

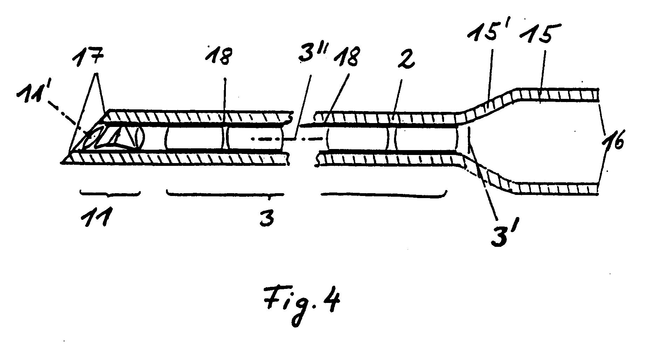

Fig. 4 zeigt ein weiteres Ausführungsbeispiel für ein erfindungsgemäßes Endoskop, das in an sich bekannter Weise ein Objektiv 11 aufweist, dessen Bild ein beispielsweise aus einer Reihe von Stablinsen sowie ggf. weiteren Linsen bestehendes Bildweiterleiter-System 3 vom distalem Ende zu einer proximalem Bildebene 3' weiterleitet. Ein Lichtleiter 2 umgibt das Objektiv 11 sowie das Relais-Linsensystem 3.

Im Gegensatz zu herkömmlichen Endoskopen besteht der Lichtleiter 2 aus einem einstückigen Hohlzylinder, an den sich bei dem gezeigten Ausführungsbeispiel ein weiterer zylindrischer Bereich 15 mit größerem Innen- und Außendurchmesser als beim Hohlzylinder 2 anschließt. Der Übergang zwischen den Zylindern 2 und 15 erfolgt kontinuierlich in einem Bereich 15'. Damit kann der Lichtleiter 2 das an der eintrittsseitigen Stirnfläche 16 des Hohlzylinders 15 eintretende Licht zur austrittsseitigen (distalen) Stirnfläche 17 leiten. Bei den gezeigten Ausführungsbeispiel ist das Objektiv ein sog. "Schrägblick-Objektiv", das zusätzlich zu den Linsen Prismen aufweist, so daß die Beobachtungsachse 11' des Objektivs 11 einen Winkel zu der optischen Achse 3'' des Relais-Linsensystems 3 und damit zur Längsachse des gezeigten Endoskop-Grundteils einschließt.

Um zu verhindern, daß Licht aus dem Lichtleiter 2 in den Bereich des Relais-Linsensystems oder in den Bereich des Objektivs 11 übertritt und dort zu Reflexionen bzw. Überstrahlungen führt, ist zwischen das Relais-Linsensystem 3 bzw. das Objektiv 11 und den Lichtleiter 2 ein lichtundurchlässiges Teil 18 eingesetzt. Dieses Teil 18 ist bei dem gezeigten Ausführungsbeispiel ein Kunststoff-Schrumpfschlauch. In diesen Kunststoff-Schrumpfschlauch werden zunächst die Linsen des Relais-Linsensystems 3 sowie ggf. die Linsen und Prismen des Objektivs 11 eingesetzt und in der richtigen Stellung positioniert. Anschließend wird der Schrumpfschlauch durch Anwendung von Wärme geschrumpft, so daß er die Linsen hält, ohne das eine Linsenfassung im herkömmlichen Sinne erforderlich wäre.

Bei dem gezeigten Ausführungsbeispiel bestehen der Lichtleiter 2 sowie ggf. die Linsen des Objektivs 11 und des Relais-Linsensystems 3 aus einem thermisch formbaren und spritzgießbaren Kunststoffmaterial, bspw. aus Polycarbonat.

Damit ist es möglich, den Lichtleiter 2 bspw. aus "Rohr-Meterware" herzustellen, wobei das Teil 15 mit größerem Durchmesser durch entsprechende thermische Formung bzw. Aufweitung hergestellt wird.

Die Herstellung der Linsen durch Spritzgießen ist wesentlich kostengünstiger als die herkömmliche Herstellung von Glaslinsen, so daß sich die Herstellkosten des erfindungsgemäßen Endoskop-Grundteils gegenüber herkömmlichen Endoskopen deutlich verringern.

In den Teil 15 mit erweitertem Durchmesser können - wie bei dem in Fig. 1 dargestellten Ausführungsbeispiel beschrieben - bspw. ein Okular oder eine Miniatur-Fernsehkamera eingesetzt werden.

Bei dem gezeigten Ausführungsbeispiel ist außen auf den Lichtleiter ein lichtundurchlässiger Lack, beispielsweise ein mit entsprechenden Fabrpigmenten versetzter Polyurethanlack aufgebracht, der u.a. den Vorteil hat, vergleichsweise Gewebe-verträglich zu sein. Weiterhin kann an dem Grundteil ein Kunststoff-Formteil mit einem in an sich bekannter Weise ausgebildeten Flanschelement zum Einsetzen in herkömliche Endoskop-Schäfte angebracht werden.

Selbstverständlich ist es aber auch möglich, den erfindungsgemäßen Endoskop-Grundteil in ein Metallschutzrohr einzubringen.

## Patentansprüche

1. Endoskop mit
- einem am distalen Ende angeordneten Endoskopobjektiv,
- einer Bildleiter-Einheit (3), die das Bild des Endoskop-objektivs zum proximalen Ende leitet, und
- einer Beleuchtungseinrichtung, die einen Lichtleiter (7) aufweist, der das Licht von einer Lichtquelle zum distalen Ende leitet, und der die Bildleiter-Einheit (3) ringförmig umgibt, sowie
- einer Videokamera (5), die an dem Endoskop abnehmbar angebracht ist, und deren Bildaufnehmer (6) das von der Bildleiter-Einheit (3) weitergeleitete Bild aufnimmt,
**gekennzeichnet** durch die Kombination folgender Merkmale:
- der Bildaufnehmer (6) der Videokamera (5) ist direkt in der proximalen Bildebene (3') der Bildleiter-Einheit (3) angeordnet,
- der Lichtleiter (7) umgibt den Bildaufnehmer (6) und ist derart ausgebildet, daß er beim Ansetzen der Videokamera (5) an das Endoskop mit einem Lichtleiter (2) des Endoskops gekoppelt wird, so daß das Licht der Beleuchtungslichtquelle in die Stirnfläche des Lichtleiters (2) des Endoskops eintritt.

2. Endoskop nach Anspruch 1,
**gekennzeichnet** durch die Kombination folgender Merkmale:
- der Lichtleiter (2) des Endoscops weist einen aus einem durchsichtigen und optisch klaren Material bestehenden Hohlzylinder auf, der das Objektiv (11) und das Relais-Linsensystem (3) umgibt,
- das Licht der Beleuchtungslichtquelle ist in die ringförmige Stirnfläche (16) des Hohlzylinders einkoppelbar.

3. Endoskop nach Anspruch 1 oder 2,
dadurch **gekennzeichnet**, daß die als Lichteintrittsfläche (16) dienende Stirnfläche in Längsrichtung (3'') des Endoskops vor oder hinter der proximalen Bildebene (3') des Relais-Linsensystems (3) bzw. des Bildaufnehmers (6) liegt.

4. Endoskop nach Anspruch 2 oder 3,
dadurch **gekennzeichnet**, daß zwischen Bildleiter-Einheit (3) und proximaler Bildebene (3') der Bildleiter-Einheit (6) ein zusätzliches optisches Element (9) vorgesehen ist, das die Bildgröße an die Größe des Bildaufnehmers (6) anpaßt.

5. Endoskop nach einem der Ansprüche 2 bis 4,
dadurch **gekennzeichnet**, daß ein Kamera-Anschlußkabel für die Videokamera (5) mit dieser mittels eines Steckers verbunden ist, in dem die Kupplung für den Lichtleiter (7') und die Verbindungselemente (11) für die elektrischen Verbindungen einen Abstand senkrecht zur Kabel-Längsachse aufweisen.

6. Endoskop nach Anspruch 5,
dadurch **gekennzeichnet**, daß im Kamera-Anschlußkabel die elektrischen Verbindungsleitungen den Lichtleiter (7') umgeben.

7. Endoskop nach einem der Ansprüche 2 bis 6,
dadurch **gekennzeichnet**, daß zusätzlich oder anstelle der Videokamera (5) mittels eines optischen Bildteilers ein Okular anbringbar ist, an dem ein an sich bekannter seitlicher Lichtleiter-Eintrittsstutzen angebracht ist, und das eine Beleuchtungslicht-Austrittsfläche gegenüber der ringförmigen Beleuchtungslicht-Eintrittsfläche des Endoskop-Grundteils aufweist.

8. Endoskop nach einem der Ansprüche 1 bis 7,
dadurch **gekennzeichnet**, daß der Hohlzylinder (2) im proximalen Bereich in einen zylindrischen Abschnitt (15) mit größerem Innen- und Außendurchmesser übergeht, dessen freie Stirnfläche (16) die Licht-Eintrittsfläche ist.

9. Endoskop nach einem der Ansprüche 1 bis 8,
dadurch **gekennzeichnet**, daß die Lichtleiter-Austrittsfläche (17) entsprechend der Beobachtungsrichtung (11') des Objektivs (11) abgeschrägt ist.

10. Endoskop nach einem der Ansprüche 1 bis 9,
dadurch **gekennzeichnet**, daß zwischen dem Hohlzylinder (2, 15) und dem Objektiv (11) bzw. dem Relais-Linsensystem (3) ein lichtabsorbierendes und/oder -reflektierendes Element (18) vorgesehen ist.

11. Endoskop nach Anspruch 10,
dadurch **gekennzeichnet**, daß das zwischen dem Hohlzylinder (2) und dem Objektiv (11) bzw. dem Relais-Linsensystem (3) vorgesehene Element (18) ein Kunststoff-Schrumpfschlauch ist, der gleichzeitig wenigstens die Elemente des Relais-Linsensystems (3) hält.

12. Endoskop nach einem der Ansprüche 1 bis 11,
dadurch **gekennzeichnet**, daß der Lichtleiter (2) des Endoskops aus einem optisch klaren Kunststoffmaterial besteht.

13. Endoskop nach einem der Ansprüche 1 bis 12,
dadurch **gekennzeichnet**, daß wenigstens die Elemente des Relais-Linsensystems (3) aus einem optisch klaren Kunststoffmaterial bestehen.

14. Endoskop nach Anspruch 12 oder 13,
dadurch **gekennzeichnet**, daß das Kunststoffmaterial Polycarbonat ist.

15. Endoskop nach einem der Ansprüche 1 bis 14,
dadurch **gekennzeichnet**, daß ein Kunststoff-Formteil mit einem in an sich bekannter Weise ausgebildeten Flanschelement zum Einsetzen in herkömliche Endoskop-Schäfte vorgesehen ist.

## Claims

1. Endoscope comprising
- an endoscope lens disposed at the distal end,
- an image-transmitting unit (3) which conveys the image from said endoscope lens to the proximal end, and
- an illuminating means comprising a light guide (7) which conveys the light from a light source to said distal end and which surrounds said image-transmitting unit (3) in an annular form, as well as
- a video camera (5) detachably disposed on the endoscope and comprising an image pickup means (6) receiving the image transmitted by said image-transmitting unit (3),
**characterized** by the combination of the following features:
- said image pickup means (6) of said video camera (5) is disposed directly in the proximal image plane (3') of said image-transmitting unit (3),
- said light guide (7) surrounds said image pickup means (6) and is configured for being coupled, by means of a light guide (2) of the endoscope, when said video camera (5) is attached to the endoscope, so that the light of said illuminating light source enters into the front side of said light guide (2) of the endoscope.

2. Endoscope according to Claim 1,
**characterized** by the combination of the following features:
- said light guide (2) of the endoscope comprises a hollow cylinder made of a transparent and optically clear material, which surrounds said lens (11) and said relay-lens system (3),
- the light of said illuminating light source may be introduced into the annular front side (16) of said hollow cylinder.

3. Endoscope according to Claim 1 or 2,
**characterized** in that said front side, which serves as light entry surface (16) is positioned, along the longitudinal direction (3'') of the endoscope, upstream or downstream of the proximal image plane (3') of said relay-lens system (3) or said image pickup means (6), respectively.

4. Endoscope according to Claim 2 or 3,
**characterized** in that an additional optical element (9) is provided between said image-transmitting unit (3) and the proximal image plane (3') of said image pickup means (6), so as to adapt the size of the image to the size of said image pickup means (6).

5. Endoscope according to any of Claims 3 to 4,
**characterized** in that a camera connecting cable for said video camera (5) is connected to the latter by means of a connector wherein the coupler for said light guide (7') and said connecting elements (11) for the electrical connections are spaced from each other in a sense normal to the longitudinal axis of the cable.

6. Endoscope according to Claim 5,
**characterized** in that the electrical connecting lines surround said light guide (7') in said camera connecting cable.

7. Endoscope according to any of Claims 2 to 6,
**characterized** in that an eyepiece may be attached by means of an optical image splitter, either additionally to or instead of said video camera (5), on which is disposed a lateral light guide entry connector known per se and which comprises an illuminating-light entry surface of the basic endoscope body.

8. Endoscope according to any of Claims 1 to 7,
**characterized** in that in the proximal zone, said hollow cylinder (2) passes over into a cylindrical portion (15) having wider inner and outer diameters, whose free front side libre (16) is the light entry surface.

9. Endoscope according to any of Claims 1 to 8,
**characterized** in that the light guide exit surface (17) is bevelled in correspondence with the direction of observation (11') of said lens (11).

10. Endoscope according to any of Claims 1 to 9,
**characterized** in that a light-absorbing and/or light-reflecting element (18) is provided between said hollow cylinder (2, 15) and said lens (11) or said relay-lens system (3), respectively.

11. Endoscope according to Claim 10,
**characterized** in that said element (18) provided between said hollow cylinder (2) and said lens (11) or said relay-lens system (3), respectively, is a shrinkable plastic hose holding, at the same time, at least the elements of said relay-lens system (3).

12. Endoscope according to any of Claims 1 to 11,
**characterized** in that said light guide (2) of the endoscope consists of an optically clear plastic material.

13. Endoscope according to any of Claims 1 to 12,
**characterized** in that at least the elements of said relay-lens system (3) are made of an optically clear plastic material.

14. Endoscope according to Claim 12 or 13,
**characterized** in that said plastic material is polycarbonate.

15. Endoscope according to any of Claims 1 to 14,
**characterized** in that a molded plastic element, including a flange part configured in a manner known per se, is provided for being inserted into conventional endoscope shafts.

## Revendications

1. Endoscope comprenant
- un objectif d'endoscope, disposé à l'extrémité distale
- une unité conductrice d'image (3), qui transmet l'image dudit objectif d'endoscope à l'extrémité proximale, et
- un dispositif d'éclairage, qui comprend un guide de lumière (7) qui transmet la lumière d'une source lumineuse à ladite extrémité distale et qui entoure ladite unité conductrice d'image (3) de façon annulaire,
- ainsi qu'une caméra vidéo (5) disposée, de façon détachable, sur l'endoscope, et comprenant un moyen de prise de vue (6) qui reçoit l'image transmise par ladite unité conductrice d'image (3),
**caractérisé** par la combinaison des caractéristiques suivantes:
- ledit moyen de prise de vue (6) de ladite caméra vidéo (5) est disposé directement dans le plan d'image proximal (3') de ladite unité conductrice d'image (3),
- ledit guide de lumière (7) entoure ledit moyen de prise de vue (6) et est configuré de façon à être accouplé par un guide de lumière (2) de l'endoscope, quand ladite caméra vidéo (5) est ajoutée à l'endoscope, d'une manière que la lumière de ladite source lumineuse d'éclairage entre dans la face dudit guide de lumière (2) de l'endoscope.

2. Endoscope selon la revendication 1,
**caractérisé** par la combinaison des caractéristiques suivantes:
- ledit guide de lumière (2) de l'endoscope comprend un cylindre creux, qui est fait d'une matière claire et optiquement transparente, qui entoure ledit objectif (11) et ledit système à lentilles en relais (3),
- la lumière de ladite source lumineuse d'éclairage peut être introduite dans la face annulaire (16) dudit cylindre creux.

3. Endoscope selon la revendication 1 ou 2,
**caractérisé** en ce que ladite face, qui fait fonction de surface d'entrée de lumière (16), est positionnée, en sens longitudinal (3'') de l' endoscope, devant ou derrière le plan d'image proximal (3') dudit système à lentilles en relais (3) ou respectivement dudit moyen de prise de vue (6).

4. Endoscope selon la revendication 2 ou 3,
**caractérisé** en ce qu'un élément optique supplémentaire (9) est prévu entre ladite unité conductrice d'image (3) et le plan d'image proximal (3') dudit moyen de prise de vue (6), afin d'adapter l'étendue de l'image à l'étendue dudit moyen de prise de vue (6).

5. Endoscope selon une quelconque des revendications 3 à 4,
**caractérisé** en ce qu'un câble de raccordement de la caméra pour ladite caméra vidéo (5) est raccordé à la dernière moyennant un connecteur dans lequel l'accoupleur pour ledit guide de lumière (7') et lesdits éléments de connexion (11) pour les connexions électriques présentent une distance orthogonale sur l'axe longitudinal du câble.

6. Endoscope selon la revendication 5,
**caractérisé** en ce que les lignes de connexion électrique entourent ledit guide de lumière (7') dans ledit câble de raccordement de la caméra.

7. Endoscope selon une quelconque des revendications 2 à 6,
**caractérisé** en ce qu'un oculaire peut être attaché moyennant un sectionneur d'image optique, additionnellement à ou au lieu de ladite caméra vidéo (5), auquel est disposé un manchon d'entrée de guide de lumière latéral, qui est connu en soi, et qui comprend une surface de sortie de la lumière d'éclairage en face de ladite surface d'entrée de la lumière d'éclairage annulaire dudit corps de base de l'endoscope.

8. Endoscope selon une quelconque des revendications 1 à 7,
**caractérisé** en ce que dans la zone proximale, ledit cylindre creux (2) se convertit en une partie cylindrique (15) aux plus grands diamètres intérieur et extérieur, dont la face libre (16) est la surface d'entrée de la lumière.

9. Endoscope selon une quelconque des revendications 1 à 8,
**caractérisé** en ce que la surface de sortie (17) dudit guide de lumière est biseautée en correspondance avec la direction d'observation (11') dudit objectif (11).

10. Endoscope selon une quelconque des revendications 1 à 9,
**caractérisé** en ce qu'un élément (18), qui absorbe et/ou réfléchit de lumière, est prévu entre ledit cylindre creux (2, 15) et ledit objectif (11) ou respectivement ledit système à lentilles en relais (3).

11. Endoscope selon la revendication 10,
**caractérisé** en ce que ledit élément (18) prévu entre ledit cylindre creux (2) et ledit objectif (11) ou respectivement ledit système à lentilles en relais (3), est une gaine rétrécissable en matière plastique, qui tient, en même temps, au moins les éléments dudit système à lentilles en relais (3).

12. Endoscope selon une quelconque des revendications 1 à 11,
**caractérisé** en ce que ledit guide de lumière (2) de l'endoscope consiste en une matière plastique optiquement transparente.

13. Endoscope selon une quelconque des revendications 1 à 12,
**caractérisé** en ce qu'au moins les éléments dudit système à lentilles en relais (3) sont faits d'une matière plastique optiquement transparente.

14. Endoscope selon la revendication 12 ou 13,
**caractérisé** en ce que ladite matière plastique est polycarbonate.

15. Endoscope selon une quelconque des revendications 1 à 14,
**caractérisé** en ce qu'un préformé en matière plastique, à un élément connecteur configuré de façon connue en soi, est prévu à être inséré dans des tiges d'endoscope conventionnelles.
